# EUROPÄISCHE PATENTSCHRIFT

(11) **EP 1 629 811 B1**
(45) Veröffentlichungstag und Bekanntmachung des Hinweises auf die Patenterteilung: **21.01.2015**
(21) Anmeldenummer: 05018283.1
(22) Anmeldetag: 23.08.2005
(51) Int. Cl.: A61F 13/06

(54) **Elastische Kniegelenkbandage**
Elastic knee joint bandage
Bandage elastique pour le genou

(30) Priorität: 23.08.2004 DE 102004040793
(43) Veröffentlichungstag der Anmeldung: 01.03.2006
(73) Patentinhaber: Bauerfeind AG, 07937 Zeulenroda (DE)
(72) Erfinder: Reinhardt, Holger, 47906 Kempen (DE); Bauerfeind, Hans B., 07937 Zeulenroda (DE); Prof. Heinrich Hess, 66271 Kleinblittersdorf (DE); Prof. Dr. Wolfgang Krause, 36145 Hofbieber (DE)
(74) Vertreter: Puschmann Borchert Bardehle Patentanwälte Partnerschaft mbB

(56) Entgegenhaltungen:
- EP-A- 0 360 277
- DE-A1- 3 416 231
- DE-A1- 3 637 879
- DE-A1- 3 838 576
- US-A- 5 730 710

## Beschreibung

Die Erfindung bezieht sich auf eine aus elastischem Textilmaterial bestehende Kniegelenkbandage, die mit einer die Kniescheibe in einer Aussparung umfassenden Profileinlage versehen ist, die von einem an dem Textilmaterial befestigten Überzug aus gleichem oder ähnlichen Textilmaterial abgedeckt ist, wobei der Profileinlage ein biegsames, nicht dehnbares Spannglied zugeordnet ist, das die Bereiche der Kniescheibenpole wadenbeinseitig im Bogen um die Kniescheibe derart verbindet, dass bei Vergrößerung der Entfernung dieser Bereiche beim Beugen des Kniegelenkes der Abstand des Bogens von der Verbindungslinie der Kniescheibenpole verringert wird und das Spannglied auf die benachbarte Seite der Kniescheibe diese medial verschiebend und zentrierend drückt.

Eine derartige Kniegelenkbandage ist in der DE 38 38 576 A1 beschrieben und dargestellt. Bei der bekannten Kniegelenkbandage wird das Spannglied in der Profileinlage geführt, was zur Folge hat, dass bei einem auf das Spannglied funktionsgemäß wirkenden Zug dieses, geführt von der Profileinlage, seine Wirkung auf die Kniescheibe ausüben kann, ohne gegenüber dieser irgendwie seitlich ausweichen zu können. Damit wird also die Profileinlage selbst in die Ausübung einer auf die Kniescheibe wirkenden Verschiebungskraft einbezogen.

Der Erfindung liegt die Aufgabe zugrunde, die Übertragung der Verschiebungskraft von dem Spannglied auf die Profileinlage überflüssig zu machen. Erfindungsgemäß geschieht dies dadurch,dass das Spannglied an dem Überzug derart befestigt ist, dass es sämtliche Veränderungen der Lage des Überzugs mitmacht und beim Beugen des Kniegelenks und Dehnung des Überzugs dieser eine Verschiebungskraft auf das Spannglied ausübt und dieses die Kniescheibe verschiebt.

Aufgrund der Befestigung des Spanngliedes an dem die Profileinlage abdeckenden Überzug übernimmt dieser als ein wesentlicher Bestandteil der Kniegelenkbandage die Führung des Spanngliedes und damit auch die Ausübung der Verschiebungskraft auf die Kniescheibe, so dass die Profileinlage ihre im Wesentlichen von dem Überzug definierte Positionierung beibehalten kann, ohne direkt von dem Spannglied bei dessen Spannung verschoben zu werden. Darüber hinaus ergibt sich mit der Kombination von Spannglied und Überzug ein wesentlicher fabrikatorischer Vorteil. Die Einbettung des Spanngliedes in die Profileinlage stellt nämlich fertigungstechnisch insofern ein Problem dar, als derartige Profileinlagen in einem Spritzvorgang ausgeformt werden, in dem dann gleichzeitig das Spannglied, integriert in das Material der Profileinlage, einbezogen werden muss. Die Befestigung des Spanngliedes direkt an dem Überzug stellt einen einfachen fabrikatorischen Vorgang dar, der beispielsweise durch Ankleben des Spanngliedes an dem Überzug bewerkstelligt werden kann, was darum fabrikatorisch unproblematisch ist, weil die betreffende Seite des Überzuges bei der Zusammenfügung der Bestandteile der Kniegelenkbandage zunächst frei zur Verfügung steht.

Für die Befestigung des Spanngliedes an dem Überzug gibt es verschiedene Möglichkeiten. Eine bevorzugte Möglichkeit besteht darin, das Spannglied auf dem Überzug aufzukleben. Daneben ist aber natürlich auch Annähen möglich.

Um beim Beugen des Knies eine für den Träger der Bandage unangenehme Faltenbildung im Kniegelenk zu vermeiden, wird zweckmäßig gegenüber der Profileinlage im Bereich der Kniekehle ein hochelastischer Einsatz in das Textilmaterial der Bandage eingearbeitet. Dieser Einsatz dehnt sich beim Strecken des Knies und zieht sich bei dessen Beugung wieder zusammen, ohne dass dabei wesentliche Falten entstehen.

Die Kniegelenkbandage lässt sich hinsichtlich ihrer Benutzbarkeit weiter dadurch verbessern, dass im Bereich zwischen Profileinlage und dem zum Fuß weisenden Rand der Bandage ein weiterer hochelastischer Einsatz in das Textilmaterial der Bandage eingearbeitet ist. Dieser hochelastische Einsatz erleichtert es, die Kniegelenkbandage über die Ferse zu ziehen, weil sich bei der dabei erforderlichen Ausdehnung der Bandage der hochelastische Einsatz dehnen lässt, wodurch dann die Bandage leicht über den Fuß und insbesondere die Ferse angelegt werden kann.

In den Figuren sind Ausführungsbeispiele der Erfindung dargestellt. Es zeigen:
- Figur 1: eine Draufsicht auf das Knie mit angelegter Kniegelenkbandage;
- Figur 2: das Knie mit angelegter Kniegelenkbandage im Schnitt gemäß der Linie II-II aus Figur 1;
- Figur 3: einen Schnitt gemäß der Linie III-III aus Figur 1;
- Figur 4: eine Seitensicht eines gebeugten Knies mit angelegter Kniegelenkbandage.

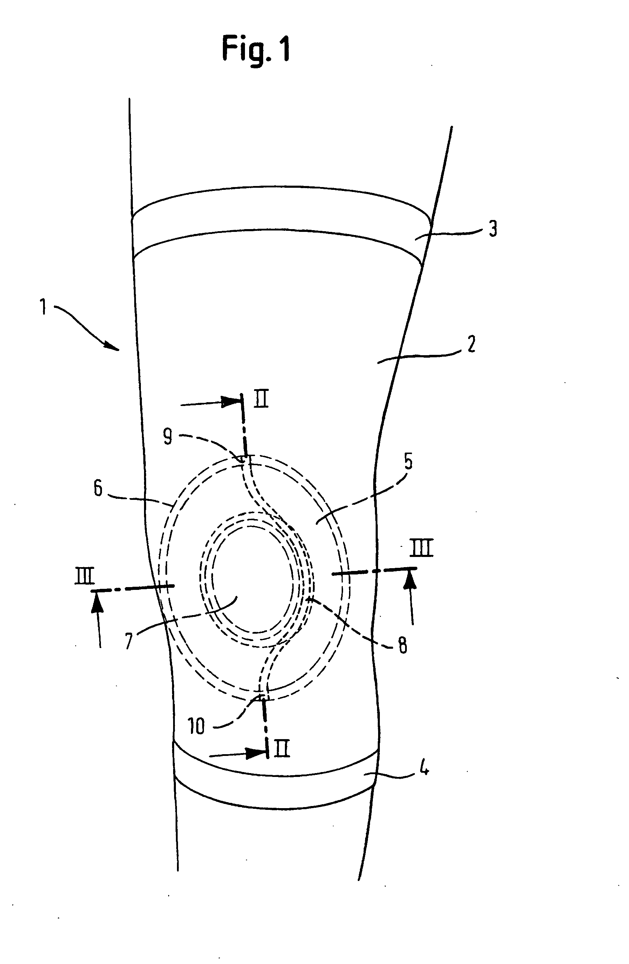

Die in der Figur 1 dargestellte Kniegelenkbandage 1 besteht aus dem aus elastischem Textilmaterial bestehenden Strumpf 2, die mit den beiden Rändern 3 und 4 an ihren beiden Enden versehen ist, die zur Rutschsicherung der Bandage 1 beitragen. Außerdem sind diese Ränder 3 und 4 aus einem Material hergestellt, das eine geringere Spannung als der Strumpf 2 aufweist, um das Bein des Trägers an den betreffenden Stellen nur wenig einzuschnüren. Auf der Vorderseite des Kniegelenks ist in dem Strumpf 2 die Profileinlage 5 eingearbeitet, die z.B. aus Schaumstoff oder Silikon bestehen kann und eine erhebliche Elastizität besitzt. Diese Profileinlage 5 ist auf der Innenseite des Strumpfes 2 durch den Überzug 6 abgedeckt, der an seinen Rändern mit dem Strumpf 2 verbunden ist, beispielsweise durch Verkleben. Die Profileinlage 5 lässt in ihrem mittleren Teil einen Bereich frei, in dem etwa die Kniescheibe 7 hinein passt. Die Kniescheibe 7 wird somit von der Profileinlage 5 umfasst. Insoweit handelt es sich um eine in bekannter Weise gestaltete Kniegelenkbandage, wie sie in der eingangs erwähnten DE 38 38 576 dargestellt ist.

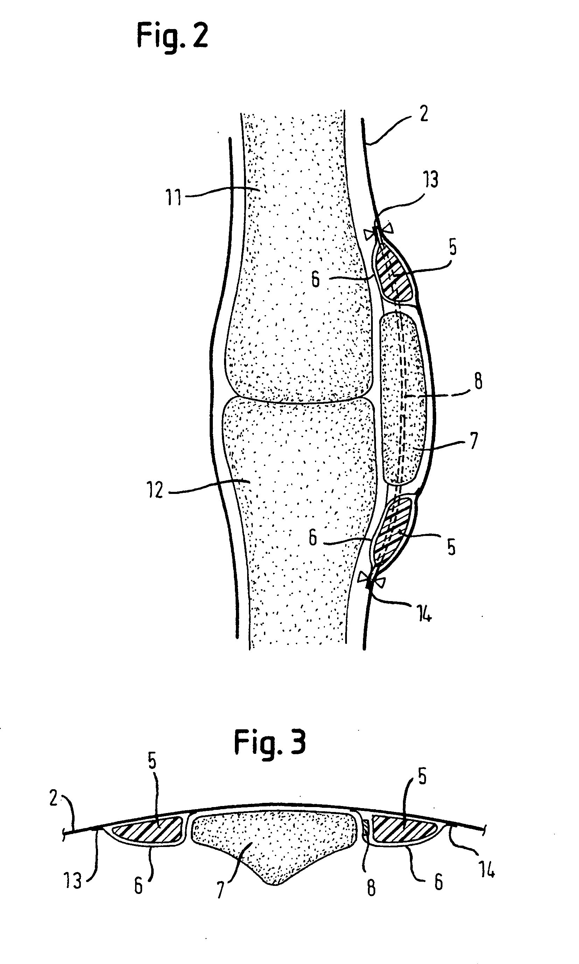

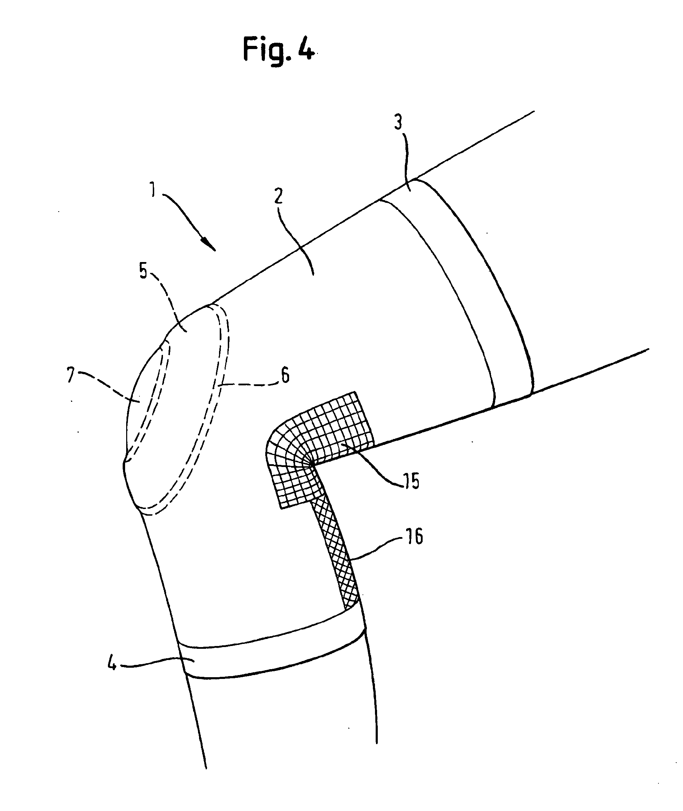

Wie auch aus den weiter unten erläuterten Schnittzeichnungen der Figuren 2 und 3 zu sehen ist, enthält der Strumpf 2 im Bereich der Profileinlage 5 das Spannglied 8, was hier als flexibler, nicht dehnbarer Textilstreifen ausgebildet ist. Das Spannglied 8 ist an dem Überzug 6 befestigt, so dass es sämtliche Veränderungen der Lage des Überzugs 6 mitmacht. Wenn das Kniegelenk gebeugt wird (siehe Figur 4), so ergibt sich dabei eine Entfernung der beiden Enden 9 und 10 des Spanngliedes 8, was zu einer Streckung der in Figur 1 dargestellten Lage des Spanngliedes 8 führt. Aufgrund dieser Streckung übt dann das Spannglied 8 auf die von der Profileinlage 5 umfasste Kniescheibe 7 einen Druck aus, der in gewünschter Weise die Kniescheibe 7 entsprechend verschiebt. Auf diesen Effekt wird in der oben erwähnten DE 38 38 576 A1 unter Bezugnahme auf deren Figuren 3 und 4 näher eingegangen.

Eine besonders vorteilhafte Befestigung des Spanngliedes 8 an dem Überzug 6 besteht darin, dass das Spannglied auf den Überzug 6 aufgeklebt ist. Dabei kann das Spannglied 8 sowohl auf der dem Bein zugewandten als auch der von dem Bein abgewandten Seite des Überzugs 6 aufgeklebt werden. Es sei aber darauf hingewiesen, dass natürlich auch eine andere Befestigungsmöglichkeit für das Spannglied besteht, z.B. durch Annähen.

In der Figur 2 ist ein Schnitt längs der Linie II-II aus Figur 1 dargestellt, in der der Oberschenkelknochen 11 und der Unterschenkelknochen 12 eingezeichnet sind, vor deren Gelenkstelle die Kniescheibe 7 gezeichnet ist. Über das Kniegelenk ist der Strumpf 2 gezogen, der das Kniegelenk allseitig umhüllt. Im Bereich der Kniescheibe 7 ist, diese umgebend, die Profileinlage 5 eingezeichnet, die an der Innenseite des Strumpfes 2 angeordnet ist und von dem Überzug 6 in Richtung zum Kniegelenk abgedeckt ist. Der Überzug 6 ist an dem Strumpf 2 ringartig befestigt. Die beiden in der Schnittdarstellung gemäß Figur 2 ersichtlichen äußeren Befestigungsstellen sind mit 13 und 14 bezeichnet. An diesen Befestigungsstellen 13 und 14 ist auch das Spannglied 8 befestigt, das über seine gesamte Länge an dem Überzug 6 befestigt ist und somit bei einem Spannen des Überzugs 6 ebenfalls mitgespannt wird. An diesen Befestigungsstellen 13 und 14 kann auch wahlweise eine Zusatzkraft, z.B. durch Gurte, ansetzen, die dem Spannglied 8 eine zusätzliche Verspannung gibt. Dabei übt es dann die im Zusammenhang mit der Darstellung zu Figur 1 erläuterten Verschiebungskräfte aus.

In der Figur 3 ist der betreffende Teil der Kniegelenkbandage 1 im Schnitt gemäß der Linie III-III dargestellt. Figur 3 zeigt den Strumpf 2 und den an ihm befestigten Überzug 6 mit den Befestigungsstellen 13 und 14 (siehe Figur 2). Der Überzug 6 schließt die ringartig geformte Profileinlage 5 ein, die ihrerseits die Kniescheibe 7 umschließt. In dem Überzug 6 ist auf dessen Innenseite das Spannglied 8 befestigt, das sich in Längsrichtung des Beins erstreckt, wie dies anhand der Figur 1 erläutert ist. Eine Spannung des Überzugs 6 aufgrund des Beugens des betreffenden Knies wird damit direkt auf das Spannglied 8 übertragen, was dann seine Verschiebungskräfte in Richtung auf die Kniescheibe 7 ausübt.

In der Figur 4 ist die Kniegelenkbandage 1, angelegt an einem gebeugten Knie, dargestellt. In den Strumpf 2 der Kniegelenkbandage ist im Bereich der Kniekehle gegenüber der Profileinlage 5 der hochelastische Einsatz 15 eingearbeitet, der den Strumpf 2 in diesem Bereich eine besondere Elastizität gibt. Durch diesen Einsatz 15 wird erreicht, dass beim Strecken und Beugen des Knies sich keine Falten im Kniegelenk bzw. beim Strecken des Knies eine besondere Spannung in dem Strumpf ergibt, so dass das Kniegelenk unbehindert gebeugt und gestreckt werden kann.

Die Kniegelenkbandage gemäß Figur 4 enthält weiterhin im Anschluss an den hochelastischen Einsatz 15 in Richtung Fuß einen weiteren hochelastischen Einsatz 16, der dazu dient, das Anziehen der Kniegelenkbandage 1, insbesondere das Überziehen über die Ferse, zu erleichtern. Bei diesem Überziehen wird der Bereich 16 besonders gedehnt, der sich dann aber aufgrund seiner hohen Elastizität nach Anlegen im Bereich des Kniegelenks wieder entsprechend zusammenzieht.

## Patentansprüche

1. Aus elastischem Textilmaterial bestehende Kniegelenkbandage (1), die mit einer die Kniescheibe (7) in einer Aussparung umfassenden Profileinlage (5) versehen ist, die von einem an dem Textilmaterial befestigten Überzug (6) aus gleichem oder ähnlichen Textilmaterial abgedeckt ist, wobei der Profileinlage (5) ein biegsames, nicht dehnbares Spannglied (g) zugeordnet ist, das die Bereiche der Kniescheibenpole wadenbeinseitig im Bogen um die Kniescheibe (7) derart verbindet, dass bei Vergrößerung der Entfernung dieser Bereiche beim Beugen des Kniegelenkes der Abstand des Bogens von der Verbindungslinie der Kniescheibenpole verringert wird und das Spannglied (8) auf die benachbarte Seite der Kniescheibe (7) diese medial verschiebend und zentrierend drückt, **dadurch gekennzeichnet, dass** das Spannglied (8) an dem Überzug (6) derart befestigt ist, dass es sämtliche Veränderungen der Lage des Überzugs (6) mitmacht und beim Beugen des Kniegelenks und Dehnung des Überzugs (6) dieser eine Verschiebungskraft auf das Spannglied (8) ausübt und dieses die Kniescheibe (7) verschiebt.

2. Kniegelenkbandage nach Anspruch 1, **dadurch gekennzeichnet, dass** das Spannglied (8) auf den Überzug (6) aufgeklebt ist.

3. Kniegelenkbandage nach Anspruch 1 oder 2, **dadurch gekennzeichnet, dass** gegenüber der Profileinlage (5) im Bereich der Kniekehle ein hochelastischer Einsatz (15) in das Textilmaterial der Bandage (1) eingearbeitet ist.

4. Kniegelenkbandage nach Anspruch 3, **dadurch gekennzeichnet, dass** im Anschluss an den hochelastischen Einsatz (15) in Richtung Fuß ein weiterer hochelastischer Einsatz (16) in das Textilmaterial der Bandage (1) eingearbeitet ist.

## Claims

1. A knee joint bandage (1) made of elastic textile material and comprising a profile insert (5) which surrounds the patella (7) in a cut-out portion, said insert being covered by a cover (6) attached to said textile material and made of the same or a similar textile material, said profile insert (5) being associated with a flexible inextensible tensioning member (g) which connects the areas of the patella poles on the side of the fibula in an arc around the patella (7) such that when the distance between these areas increases as the knee joint is being bent, the distance of the arc from the connection line of the patella poles will be diminished and that said tensioning member (8) will press on the adjacent side of the patella (7) so as to medially displace as well as center the latter **characterized in that** said tensioning member (8) is attached to said cover (6) in such a way that it will move along with all the changes in position of said cover (6), and that, as the knee joint is being bent and the cover (6) is being extended, said cover (6) will exert a displacing force on the tensioning member (8), causing the latter to displace the patella (7).

2. The knee joint bandage of claim 1 **characterized in that** said tensioning member (8) is glued onto said cover (6).

3. The knee joint bandage of claims 1 or 2 **characterized in that** opposite said profile insert (5), a highly elastic insert (15) has been included in the textile material of said bandage (1) in the region of the hollow of the knee.

4. The knee joint bandage of claim 3 **characterized in that** following said first highly elastic insert (15), another highly elastic insert (16) has been included in the textile material of said bandage (1) in the direction of the foot.

## Revendications

1. Bandage pour le genou (1) en matière textile élastique pourvu d'un insert profilé (5) qui recouvre la rotule (7) et qui est lui-même recouvert d'un revêtement (6) fixé à la matière textile composé de la même ou d'une autre matière textile similaire, un élément de précontrainte souple et non extensible (8) étant assigné à l'insert profilé (5) et reliant la zone des pôles de la rotule du côté du péroné dans l'arc autour de la rotule (7) de telle façon que, lorsque l'écart augmente avec cette zone quand l'articulation du genou est pliée, la distance séparant l'arc de la ligne de liaison avec les pôles de la rotule est réduit et l'élément de précontrainte (8) pousse en direction médiale et de façon centrée sur la rotule (7), **caractérisé en ce que** l'élément de précontrainte (8) est fixé au revêtement (6) de façon à être entraîné par l'ensemble des points d'ancrage du revêtement (6) et, lorsque l'articulation du genou est pliée et le revêtement (6) allongé, ce dernier exerce une force de poussée sur l'élément de contrainte (8) et que celui-ci pousse sur la rotule (7).

2. Bandage pour le genou selon la revendication 1, **caractérisé en ce que** l'élément de précontrainte (8) est collé au revêtement (6).

3. Bandage pour le genou selon l'une des revendications 1 ou 2, **caractérisé en ce qu'**un élément hautement élastique (15) est intégré à la matière textile du bandage (1) face à l'insert profilé (5) dans la zone du creux du genou.

4. Bandage pour le genou selon la revendication 3, **caractérisé en ce que**, en plus de l'élément hautement élastique (15), un autre élément hautement élastique (16) est intégré à la matière textile du bandage (1) en direction du pied.
